Europäisches Patentamt

European Patent Office

Office européen des brevets

(11) Veröffentlichungsnummer: **0 347 463**
**A1**

(12) **EUROPÄISCHE PATENTANMELDUNG**
Veröffentlicht nach Art. 158 Abs. 3 EPÜ

(21) Anmeldenummer: 88903122.5

(22) Anmeldetag: 23.12.87

Daten der zugrundeliegenden internationalen Anmeldung:

(86) Internationale Anmeldenummer:
PCT/SU87/00150

(87) Internationale Veröffentlichungsnummer:
WO89/05790 (29.06.89 89/14)

(51) Int. Cl.³: **C 07 C 69/732**
**C 07 C 67/02**

(43) Veröffentlichungstag der Anmeldung:
27.12.89 Patentblatt 89/52

(84) Benannte Vertragsstaaten:
**BE DE FR GB IT NL**

(71) Anmelder: NAUCHNO-ISSLEDOVATELSKY INSTITUT
KHIMIKATOV DLYA POLIMERNYKH MATERIALOV
ul. Montazhnikov, 3,
Tambov, 392680(SU)

(72) Erfinder: GLUSHKOVA, Ljudmila Vasilievna
ul. Michurinskaya, 143-80
Tambov, 392032(SU)

(72) Erfinder: BELOVA, Svetlana Jurievna
2-i Pochtovy proezd, 3-51
Tambov, 392028(SU)

(72) Erfinder: PARAMONOV, Vladimir Ivanovich
ul. Michurinskaya, 143-8
Tambov, 392032(SU)

(72) Erfinder: GORBUNOV, Boris Nikolaevich
ul. Stepana Razina, 11/13-9
Tambov, 392000(SU)

(72) Erfinder: RUBINSHTEIN, Boris Iosifovich
ul. Internatsionalnaya, 65-22
Tambov, 392000(SU)

(74) Vertreter: von Füner, Alexander, Dr. et al,
Patentanwälte v. Füner, Ebbinghaus, Finck Mariahilfplatz
2 & 3
D-8000 München 90(DE)

(54) **VERFAHREN ZUR HERSTELLUNG VON PENTAERYTHRYL-TETRAKIS-3-(3,5-DITERT.BUTYL-4-OXYPHENYL)PROPIONATEN.**

(57) Verfahren zur Herstellung von Pentaerythryl-tetrakis-[3-(3,5-ditert.butyl-4-oxyphenyl) propionat] durch die Veresterung des 3- (3,5-ditert.Butyl-4-oxyphenyl) propionsäuremethylesters mit Pentaerythrit in Gegenwart eines Alkalikatalysators bei einer Temperatur von 115 bis 160°C und einem Druck von 0,66 bis 13,3 kPa. Als Alkalikatalysator wird ein Gemisch aus Kaliumkarbonat, Natriumkarbonat und Phenol bei deren Masserverhältnis von 1:1-2:2-3 entsprechend ausgenutzt, dabei wird der Katalysator in einer Menge von 6 bis 10%, bezogen auf die Masse des Ausgangsesters, verwendet. Das gewonnene Produkt ist ein hocheffektives untoxisches Antioxydationsmittel für Polymerstoffe.

EP 0 347 463 A1

# VERFAHREN ZUR HERSTELLUNG VON PENTAERYTHRYL-TETRAKIS-[3-(3,5-DITERT.BUTYL-4-OXYPHENYL)PROPIONAT]

## Technisches Gebiet

Die Erfindung bezieht sich auf das Gebiet der organischen Synthese und betrifft insbesondere Verfahren zur Herstellung von Pentaerythryl-tetrakis-[3-(3,5-ditert.butyl-4-oxyphenyl)propionat]. Das genannte Produkt ist ein hocheffektives untoxisches Antioxydationsmittel für Polyolefine, Polystyrol und andere Polymerstoffe, das die genannten Polymere nicht färbt.

## Zugrundeliegender Stand der Technik

Es ist ein Verfahren zur Herstellung von Pentaerythryl-tetrakis-[3-(3,5-ditert.butyl-4-oxyphenyl)propionat] durch die Veresterung des 3-(3,5-ditert.Butyl-4-oxyphenyl)propionsäuremethylesters mit Pentaerythrit in Gegenwart eines Alkalikatalysators - der Lithiumhydride - bei einer Temperatur von 185 bis 190°C und dem atmosphärischen Druck innerhalb von 13 Stunden unter der kontinuierlichen Entfernung des sich bei der Reaktion bildenden Methanols durch einen Stickstoffstrom oder in Gegenwart des gleichen Katalysators unter Zugabe von Dimethylsulfoxyd bei einer Temperatur von 85 bis 90°C und einem Druck von 2 bis 2,66 kPa bis zum Aufhören der Methanolausscheidung bekannt (US, A, 3644482).

Im angegebenen Verfahren wird als Alkalikatalysator Lithiumhydrid ausgenutzt, das im Verlauf der Reaktion sowie durch die in Ausgangsreagenzien und in der umgebenden Atmosphäre enthaltene Feuchtigkeit unter Wasserstoffausscheidung zersetzt wird, was die Explosions- und Feuergefahr des Prozesses vergrössert. Ausserdem wird das bekannte Verfahren durch einen langwierigen Verlauf der Veresterungsreaktion gekennzeichnet.

Es ist auch ein Verfahren zur Herstellung von Pentaerythryl-tetrakis-[3-(3,5-ditert.butyl-4-oxyphenyl)propionat] durch die Veresterung des 3-(3,5-ditert.Butyl-4-oxyphnnyl)propionsäuremethylesters mit Pentaerythrit in Gegenwart eines Alkalikatalysators u.z. einer Alkalimetallalkoxydes, beispielsweise Natriummethylat, Lithiumme - thylat bei einer Temperatur von 80 bis 200°C und einem Druck, der wenigstens ebenso niedrig ist, wie der Dampfdruck des sich bei der Reaktionstemperatur bildenden Methanols ist, bekannt. So kann man beispielsweise den Veresterungsprozeß unter Anwendung von Natriummethylat als Katalysator zuerst bei einer Temperatur von 150°C und beim atmosphärischen Druck unter Stickstoffzufuhr innerhalb von 3 Stunden und dann bei einer Temperatur von 150°C und einem Druck von 2,66 kPa innerhalb von 17 Stunden verwirklichen. Die Ausbeute an Endprodukt nach dem angegebenen Verfahren beträgt 71 bis 80% der Theorie (GB, C, 1081789).

Im gegebenen Verfahren verwendet man als Alkalikatalysator ein Alkalimetallalkoxyd, das gegen die Feuchtigkeit in Ausgangsreagenzien und in der umgebenden Atmosphäre äußerst empfindlich ist, sich unter Feuchtigkeitseinwirkung zersetzt, was dessen Aufbewahrung und Anwendung erschwert. Außerdem wird dieses Verfahren durch einen langwierigen Verlauf der Veresterungsreaktion (20 Stunden) gekennzeichnet.

Offenbarung der Erfindung

Der Erfindung liegt die Aufgabe zugrunde, in einem Verfahren zur Herstellung von Pentaerythryl- tetrakis-[3-(3,5-ditert.butyl-4-oxyphenyl)propionat] solch einen Alkalikatalysator auszuwählen, der gegen die Feuchtigkeitseinwirkung und folglich bei der Aufbewahrung beständig, bei der Anwendung gefahrlos ist, sowie es gestattete, die Dauer des Veresterungsprozesses zu kürzen.

Diese Aufgabe wird dadurch gelöst, daß ein Verfahren zur Herstellung von Pentaerythryl- tetrakis-[3-(3,5-ditert.butyl-4- oxyphenyl)propionat] durch die Veresterung des 3-(3,5-ditert.Butyl-4-oxyphenyl)propionsäuremethylesters mit Pentaerythrit in Gegenwart eines Alkalikatalysators bei einer Temperatur von 115 bis 160°C und einem Druck von 0,66 bis 13,3 kPa vorgeschlagen wird, bei dem erfindungsgemäß als Alkalikatalysator ein Gemisch aus Kaliumkarbonat, Natriumkarbonat und Phenol bei deren Masseverhältnis von 1:1 bis 2 : 2 bis 3 entsprechend ausgenutzt wird, wobei der genannte Alkalykatalysator in
einer Menge von 6 bis 10%, bezogen auf die Masse des
3-(3,5-ditert.Butyl-4-oxyphenyl)propionsäuremethylesters,
verwendet wird.

Der im erfindungsgemäßen Verfahren auszunutzende
Alkalykatalysator zersetzt sich unter Einwirkung der in
den Ausgangsreagenzien und in der umgebenden Atmosphäre
enthaltenden Feuchtigkeit nicht und ist folglich bei
einer längeren Aufbewahrung beständig, bei der Anwendung
gefahrlos (das heißt explosions- und feuerungsgefährdet).
Außerdem gestattet dieser Katalysator es die Veresterung
zu intensivieren, indem er bis auf 9 Stunden gekürzt wird.
Die technologische und apparative Gestaltung des Verfahrens ist einfach, es sichert die Herstellung des Endproduktes von einer hohen Qualität mit einer Ausbeute von
83% der Theorie, bezogen auf den Ausgangsmethylester.

Wie oben gesagt wurde, wird die Veresterung bei
einer Temperatur von 115 bis 160°C verwirklicht. Es ist
nicht zweckmäßig, den genannten Prozeß bei einer Temperatur von unter 115°C durchzuführen, weil in diesem Fall
die Veresterung mit einer geringen Geschwindigkeit vor
sich geht. Es wird nicht empfohlen, den genannten Prozeß
bei einer Temperatur von über 160°C durchzuführen, weil
in diesem Fall eine intensive Verharzung der Produkte beginnt sowie der Energieaufwand steigert.

Die Veresterung wird unter einem erniedrigten Druck
von 0,66 bis 13,3 kPa durchgeführt. Es wird nicht empfohlen, den Prozeß bei einem Druck von unter 0,66 kPa zu
verwirklichen, weil sich in diesem Fall bei der Reaktionstemperatur die Verluste am Ausgangs-3-(3,5-ditert.Butyl-
4-oxyphenyl)propionsäuremethylester infolge dessen Entfernung        - ansteigen. Es ist nicht zweckmäßig, den
Prozeß bei einem Druck von über 13,3 kPa zu verwirklichen,
weil in diesem Fall die Geschwindigkeit der Entfernung
des sich bildenden Methanols aus der Reaktionszone stark
sinkt und folglich sich die Dauer des Veresterungsprozesses verlängert.

Im erfindungsgemäßen Verfahren wird als Alkalikatalysator ein Gemisch aus Kaliumkarbonat, Natriumkarbonat und Phenol bei deren Masseverhältnis von 1:1-2:2-3 entsprechend, verwendet. Es empfiehlt sich nicht, einen Alkalikatalysator auszunutzen, in welchem die genannten Komponenten ein Masseverhältnis von unter 1:1 bzw. 2 haben, weil in diesem Fall kein positiver Effekt erreicht wird. Eine Erhöhung des Masseverhältnisses der Komponenten im Katalysator von über 1:2 bzw. 3 führt zur Herabsetzung der Aktivität des Katalysators.

Der angegebene Alkalikatalysator wird in einer Menge von 6 bis 10%, bezogen auf die Masse des 3-(3,5-ditert. Butyl-4-oxyphenyl)propionsäuremethylesters, verwendet. Es ist nicht zweckmäßig, den Katalysator in einer Menge von unter 6%, bezogen auf die Masse des Ausgangsesters, zu verwenden, weil sich in diesem Fall die Effektivität der Katalysatorwirkung vermindert. Die Verwendung des Katalysators in einer Menge von über 10%, bezogen auf die Masse des Ausgangsesters, ergibt keinen positiven Effekt und führt zur Erhöhung des Verbrauchs am Katalysator.

Beste Ausführungsvariante der Erfindung

Das erfindungsgemäße Verfahren zur Herstellung von Pentaerythryl-tetrakis-[3-(3,5-ditert.butyl-4-oxyphenyl)-propionat] wird folgenderweise verwirklicht.

In einem mit einem Rührwerk, einem Thermometer und einem System zur Erzeugung eines erniedrigten Drucks versehenen Reaktor werden 3-(3,5-ditert.Butyl-4-oxyphenyl)-propionsäuremethylester, Pentaerythrit und ein Alkalikatalysator aufgegeben, der in einer Menge von 6 bis 10%, bezogen auf die Masse des Ausgangsesters, genommen wird. Der genannte Katalysator stellt ein Gemisch aus Kaliumkarbonat, Natriumkarbonat und Phenol bei deren Masseverhältnis von 1:1-2:2-3 entsprechend dar. Im Reaktor wird ein erniedrigter Druck von 13,3 kPa erzeugt, das Ausgangsgemisch wird auf eine Temperatur von 155°C unter Rühren erwärmt und der Veresterungsprozeß wird vorzugsweise innerhalb von 9 Stunden bei einer allmählichen Temperatursteigerung bis auf 160°C und einer Drucksenkung bis auf 0,66 kPa durchgeführt. Nach der Beendigung des Veresterungsprozesses wird das Endprodukt aus der Reaktionsmasse nach bekannten Verfahren isoliert. Beispielsweise wird die Reaktionsmasse auf eine Temperatur von 100°C abgekühlt, in Benzin gelöst, vom Katalysator abfiltriert und das Zielprodukt wird aus der Benzinlösung bei einer Temperatur von 15 bis 20°C kristallisiert. Der ausgeschiedene kristalline Niederschlag des Endproduktes wird abfiltriert, wiederholt aus Benzin kristallisiert, abfiltriert und getrocknet. Die Ausbeute an Zielprodukt beträgt 78 bis 83% der Theorie, bezogen auf den Ausgangsmethylester.

Nach dem beschriebenen Verfahren wird das Endprodukt von einer hohen Qualität mit einem Schmelzpunkt von 121 bis 123°C gewonnen.

Zum besseren Verständnis der vorliegenden Erfindung werden folgende konkrete Ausführungsbeispiele angeführt.

Beispiel 1

In einen mit einem Rührwerk, einem Thermometer und einem System zur Erzeugung eines erniedrigten Drucks versehenen Reaktor werden 46,8 g (0,16 Mol) 3-(3,5-ditert. Butyl-4-oxyphenyl)propionsäuremethylester, 5,45 g (0,04 Mol) Pentaerythrit und 2,81 g (6%) bezogen auf die Masse des Ausgangsesters) Alkalikatalysator aufgegeben, der aus Kaliumkarbonat, Natriumkarbonat und Phenol bei deren Masseverhältnis von 1:2 bzw. 3 besteht. Im Reaktor wird ein erniedrigter Druck von 13,3 kPa erzeugt, das Ausgangsgemisch wird auf eine Temperatur von 115°C unter Rühren erwärmt und die Veresterung wird innerhalb von 9 Stunden unter einer allmählichen Temperatursteigerung bis auf 160°C und einer Drucksenkung bis auf 0,66 kPa durchgeführt. Nach der Beendigung des Veresterungsprozesses wird die Reaktionsmasse zur Isolierung des Endproduktes bis auf 100°C abgekühlt, in 55 cm$^3$ Benzin gelöst, vom Katalysator abfiltriert und das Endprodukt wird aus der Benzinlösung bei einer Temperatur von 18°C kristallisiert.

Der ausgeschiedene kristalline Niederschlag des Endproduktes wird abfiltriert, aus Benzin wiederholt kristallisiert, abfiltriert und getrocknet.

Man erhält 36,6 g (78% der Theorie, bezogen auf den Ausgangsester) Endprodukt in Form von weißen Kristallen mit einem Schmelzpunkt von 122 bis 123°C.

Beispiel 2

Die Herstellung von Pentaerythryl-tetrakis-[3-(3,5-ditert.butyl-4-oxyphenyl)propionat] und dessen Isolierung aus der Reaktionsmasse werden ähnlich wie im Beispiel 1 verwirklicht. Dabei wird der Alkalikatalysator in einer Menge von 4,68 g (10% von der Masse des Ausgangsesters) verwendet. Der Alkalikatalysator stellt ein Gemisch aus Kaliumkarbonat, Natriumkarbonat und Phenol bei deren Masseverhältnis von 1:1 bzw. 2 dar.

Man erhält 39,1 g (83,5% der Theorie) Endprodukt in Form von weißen Kristallen mit einem Schmelzpunkt von 121 bis 122°C.

Beispiel 3

Die Herstellung von Pentaerythryl-tetrakis-[3-(3,5-ditert.butyl-4-oxyphenyl)propionat] und dessen Isolierung aus der Reaktionsmasse werden ähnlich wie im Beispiel 1 verwirklicht. Dabei wird der Alkalikatalysator in einer Menge von 3,74 g (8%, bezogen auf die Masse des Ausgangsesters) verwendet. Der Alkalikatalysator stellt ein Gemisch aus Kaliumkarbonat, Natriumkarbonat und Phenol bei deren Masseverhältnis von 1:1,5 bzw. 2,5 dar.

Man erhält 38,8 g (83% der Theorie) Endprodukt in Form von weißen Kristallen mit einem Schmelzpunkt von 121 bis 122°C.

Der im erfindungsgemäßem Verfahren zu verwendende Alkalikatalysator zeichnet sich somit durch die Beständigkeit gegen die Feuchtigkeitseinwirkung, die Stabilität bei einer längeren Aufbewahrung, die Explosions- und Feuergefahrlosigkeit aus. Die Ausnutzung des gegebenen Katalysators gestattet es, den Veresterungsprozeß zu intensivieren, indem dieser bis auf 9 Stunden gekürzt wird.

- 7 -

### Industriemäßige Verwendbarkeit

Die vorliegende Erfindung kann auf dem Gebiet der organischen Synthese zur Herstellung von Pentaerythryl-tetrakis- [3-(3,5-ditert.butyl-4-oxyphenyl)propionat] Verwendung finden, das ein hocheffektives untoxisches Antioxydationsmittel für Polyolefine, Polystyrol und andere Polymerstoffe ist, das die genannten Polymere nicht färbt.

## PATENTANSPRUCH

Verfahren zur Herstellung von Pentaerythryl-tetrakis-[3-(3,5-ditert.butyl-4-oxyphenyl)propionat] durch die
Veresterung des 3-(3,5-ditert.Butyl-4-oxyphenyl)propionsäuremethylesters mit Pentaerythrit in Gegenwart eines
Alkalikatalysators bei einer Temperatur von 115 bis 160°C
und einem Druck von 0,66 bis 13,3 kPa, dadurch  g e -
k e n n z e i c h n e t , daß als Alkalikatalysator ein
Gemisch aus Kaliumkarbonat, Natriumkarbonat und Phenol
bei deren Masseverhältnis 1:1-2:2-3 entsprechend  verwendet wird, wobei      der        Alkalikatalysator
in einer Menge von 6 bis 10%, bezogen auf die Masse des
3-(3,5-ditert.Butyl-4-oxyphenyl)propionsäuremethylesters,
eingesetzt wird.

# INTERNATIONAL SEARCH REPORT

0347463

International Application No PCT/SU 87/00150

## I. CLASSIFICATION OF SUBJECT MATTER (if several classification symbols apply, indicate all) •

According to International Patent Classification (IPC) or to both National Classification and IPC

$IPC^4$ – C 07 C 69/732,67/02

## II. FIELDS SEARCHED

### Minimum Documentation Searched [7]

| Classification System | Classification Symbols |
|---|---|
| $IPC^4$ | C 07 C 69/732, 67/02 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| Y | SU, A1, 270725, (V. Ya. Kiselev et al.) 3 September 1970 (03.09.70), see the claims | 1 |
| Y | EP, B1, 0032459, (MITSUI PETROCHEMICAL IN- DUSTRIES, LTD.) 28 September 1983 (28.09.83), see claims 4,5 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance, the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 15 August 1988 (15.08.88) | 1 September 1988 (01.09.88) |

| International Searching Authority | Signature of Authorized Officer |
|---|---|
| ISA/SU | |

Form PCT/ISA/210 (second sheet) (January 1985)